(19) Europäisches Patentamt
European Patent Office
Office européen des brevets

(11) Publication number: **0 137 600**
**A1**

(12) **EUROPEAN PATENT APPLICATION**

(21) Application number: 84304926.3

(22) Date of filing: 19.07.84

(51) Int. Cl.⁴: C 07 D 489/04
A 61 K 31/485

(30) Priority: 20.07.83 GB 8319546

(43) Date of publication of application:
17.04.85 Bulletin 85/16

(84) Designated Contracting States:
AT BE CH DE FR GB IT LI LU NL SE

(71) Applicant: Euroceltique SA
122 Boulevard De La Petrusse
Luxembourg(LU)

(72) Inventor: Stuart, James Fraser Bruce
204 George Street
Glasgow G1 1XW Scotland(GB)

(72) Inventor: Florence, Alexander Taylor
204 George Street
Glasgow G1 1XW Scotland(GB)

(72) Inventor: Ferguson, Ruth
204 George Street
Glasgow G1 1XW Scotland(GB)

(74) Representative: Lamb, John Baxter et al,
Marks & Clerk 57/60 Lincoln's Inn Fields
London WC2A 3LS(GB)

(54) **Pharmaceutically active salts of morphine.**

(57) Pharmaceutically active salts of morphine are salts of morphine with an acid of the formula:

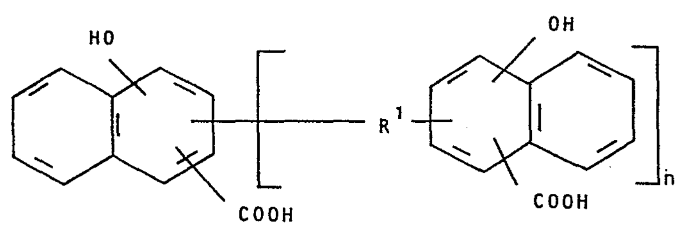

in which $R^1$ is a lower alkylene radical and *n* is 0 or 1.

The invention also provides pharmaceutical compositions the salts together with a pharmaceutically acceptable carrier or diluent.

EP 0 137 600 A1

Croydon Printing Company Ltd.

0137600
TITLE MODIFIED
see front page



Folio: 230P45053 WANGDOC:0031i

# Pharmaceutically active salts

This invention is concerned with improvements in and relating to pharmaceutically active salts of morphine, the preparation thereof, and pharmaceutical compositions containing them.

As is well known, it is often desirable that a pharmaceutically active material be administered to a patient in a form such that the interval between individual doses is not too short and, to this end, a wide variety of sustained release dosage forms have been proposed.

One measure of the effective life of a pharmaceutically active compound involves, of course, direct measurement, over a period of time, of the pharmaceutical activity of the compound administered to a test subject. Another method of the effective life of a pharmaceutically compound administered to a test subject is the concentration of the material in the blood serum of the test subject over a period of time after adminstration of the pharmaceutically active material.

It has now been found, in accordance with the present invention, that certain new salts of morphine, as hereinafter defined, give markedly extended periods of time, after administration, during which the active material has activity or is present, at significant levels, in the blood serum; as is evidenced by in vivo tests in animal and human test subjects.

Accordingly, one embodiment of the invention provides, as new compounds, salts of morphine with acids of the general formula:

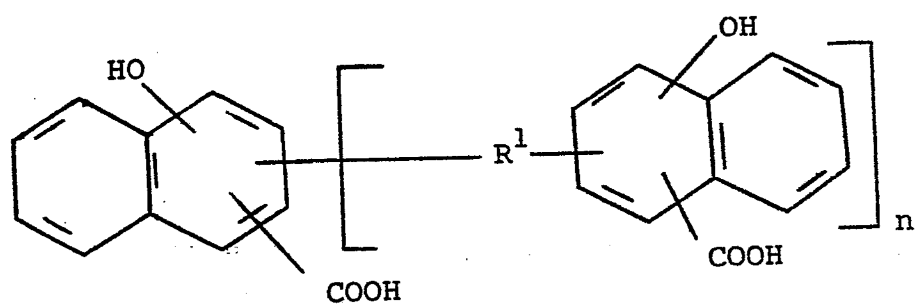

in which $R^1$ is a lower alkylene chain, preferably a methylene chain, and n is 0 or 1.

Examples of such acids include 1-hydroxy-2-naphthoic acid, 2-hydroxy-1-naphthoic acid and pamoic acid, the latter being preferred. When the acid of formula I is a dicarboxylic acid (for example pamoic acid), the salt may be a mono or di salt. Thus, particular mention may be made of dimorphine pamoate and monomorphine pamoate.

The salts of the invention are conveniently prepared by reacting the acid, or an alkali metal salt thereof, with morphine or a salt thereof with a mineral acid such as hydrochloric or sulphuric acid. The reaction is conveniently carried out, when the acid is a monocarboxylic acid, using substantially equivalent amounts of of the two reactants and, as will be appreciated, when the acid is a dicarboxylic acid, the reaction may be carried out using substantially two equivalents of morphine per equivalent of diacid (to produce the di-salt) or substantially one equivalent of morphine per equivalent of di-acid (to produce the monosalt).

Since the salts of the invention have generally low solubilities in water, the reaction between the acid (or salt thereof) and the morphine (or salt thereof) is conveniently carried out in the presence of water as a reaction medium although, as will be appreciated, the reaction may also be carried out in the presence of organic liquids as reaction mixture, typically volatile organic solvents such as ethanol.

One particular method for the preparation of the salts according to the invention (method A) involves the reaction of morphine hydrochloride with an alkali metal (generally sodium) salt of the desired acid. In this method, the morphine hydrochloride and sodium salt of

the acid are dissolved in separate quantities of water to form concentrated solutions. Each solution is filtered before the two solution are mixed together and, after mixing, the mixture is stirred mechanically for about 30 minutes, then refrigerated (for example at 2 - 10°C) overnight.

Another particular method for the preparation of the salts according to the invention (method B) involves the reaction of morphine hydrochloride with the acid itself. In this method the required amount of morphine hydrochloride is dissolved in water to form a concentrated solution and then filtered. A small excess of the required amount of acid is suspended in water and stirred. A 1N sodium hydroxide solution is then added dropwise to this suspension until it dissolves. The pH of the solution is then adjusted with 0.1 N hydrochloric acid to bring its pH to as close to 7.0 practicable (i.e. without the precipitation of the acid form of the acid). The solution is then filtered before it is added to the morphine hydrochloride solution. The resultant mixture is then stirred for about 30 minutes and then refrigerated (for example at 2 to 10°C) overnight.

The reaction product is, in each case, separated from the reaction medium by filtration, suitably by filtration through a fixed sintered glass filter under reduced pressure, and is then washed with water. The

product thus obtained is dried in a vacuum oven, suitably at room temperature over phosphorus pentoxide.

In accordance with another embodiment of the invention the acid (or salt thereof) may be reacted not with morphine per se but with the morphine-containing product opium, in which case the final reaction product may contain, in addition to the morphine salt, salts of other opium alkaloids of the phenanthene class, e.g. codeine and thebaine, and of the benzylisoquinoline class, e.g. papaverene and norscapine.

The invention also provides a pharmaceutical composition comprising a morphine salt as defined above in association with a pharmaceutically acceptable carrier or diluent. In accordance with a particularly preferred embodiment of the invention, such a composition is formulated for oral administration and thus, for example, may take the form of a suspension of the salt in a suitable carrier such as water (possibly with the addition of pharmaceutically acceptable suspending agents). Such a suspension may be administered per se or may be encapsulated. Further, the pharmaceutical compositions of the invention for oral administration may take conventional orally administrable dosage unit forms such as tablets. In order to further extend the duration of therapeutic activity of the salts the pharmaceutical compositions of the invention may take sustained release form.

Alternatively, the pharmaceutical compositions of the invention may take the form of pessaries or suppositories comprising the active salts together with an appropriate pessary or suppository base. The pharmaceutical compositions of the invention may also take a form suitable for administration by injection, i.e. comprising a suspension of the active salt in a sterile injectably administrable vehicle such as water or an oil. Finally, the pharmaceutical compositions of the invention may be put up in a form suitable for transmucosal or transdermal administration.

The morphine salts of the invention exhibit an enhanced duration of analgesic activity as compared with morphine hydrochloride when orally administered to rats and exhibit a remarkably extended period of time during which morphine is present, at significant levels, in the blood serum of human volunteers to which the salts have been orally administered. Thus, in the case of dimorphine pamoate, markedly enhanced bioavailability of the salt (as compared with morphine sulphate) was noted.

In order that the invention may be well understood the following examples are given by way of illustration only.

## EXAMPLE 1

Dimorphine pamoate trihydrate - Method A

7.516 Grams (0.1 mole) of morphine hydrochloride and 4.323 mg (0.05 mole) of sodium pamoate were each separately dissolved in the minimium quantity of water to form a concentrated solution and each solution was filtered. The filtered solutions were then mixed together and the mixture was stirred for 30 minutes and then refrigerated (at 2 - 10°C) overnight. The solid precipitate formed was filtered off from the refrigerated mixture under vacuum on a sintered glass filter. The precipitate was then washed with water and dried in a vacuum oven over phosphorous pentoxide at room temperature. The product had the following elemental analysis:

| | Found | Calculated |
|---|---|---|
| C(%) | 66.39 | 67.58 |
| H(%) | 5.92 | 5.97 |
| (N(%) | 2.442. | 2.76 |

The product had a solubility in water at 25°C of 3.0 x $10^{-3}$ mole/litre.

## EXAMPLE 2

### Morphine 3-hydroxy-2-naphthoate (Method B)

3.75 Grams (0.1 mole) of morphine hydrochloride trihydrate were dissolved in the minimum quantity of water (ca. 100 ml) and this solution was filtered.

1.88 Grams (0.1 mole) of 3-hydroxy-2-naphthoic acid were suspended in about 100 ml of water and the suspension was stirred whilst 1N aqueous sodium hydroxide was added dropwise until the suspended acid dissolved. The pH of the solution was then adjusted, by the addition of 0.1N aqueous hydrochloric acid, to a pH as close to pH 7 as practicable, i.e. without precipitation of a solid. The solution was then filtered and mixed with the aqueous morphine hydrochloride solution. The mixture was stirred for 30 minutes and then refrigerated (at 2-10°C) overnight. The solid precipitate which formed was filtered off under vacuum using a sintered glass filter, thoroughly washed with water and dried in a vaccum oven over phosphorous pentoxide at room temperature to give a buff coloured powder having the following elemental analysis:

| | Found | Calculated |
|---|---|---|
| C(%) | 68.5 | 71.0 |
| H(%) | 6.01 | 5.75 |
| N(%) | 2.95 | 2.96 |

The product had a melting point of 133-138°C and a solubility in water at 25°C of 4.67 x $10^{-3}$ mole/litre.

Pharmacological Tests

In these tests reference will be made to the accompanying drawings in which:

Figure 1 is a graph showing the analgesic activity, in rats, of morphine hydrochloride (filled circles), morphine 3-hydroxy-2-naphthoate (open diamonds) and morphine pamoate (open circles);

Figure 2 is a graph showing the analgesic activity, in rats of morphine pamoate; and

Figure 3 is a graph showing the blood serum levels, in human volunteers, after administration of morphine sulphate (filled circles) and morphine pamoate (open circles).

The analgesic activity of morphine pamoate and morphine 3-hydroxy-2-napthoate in rats was assessed using an "Analgesy-meter" (Ugo Basile, Italy). The rats employed in a tests were male Wistar rats having a weight of 180 - 200 grams and in each of the tests batches of three rats were employed.

The "Analyesy ß" is an apparatus designed to estimate the analgesic activity of a material by a

pressure method in which the gradual and steadily increasing pressure is applied to the test animals' tail by means of a cone-shaped pusher with a rounded tip. In the tests reported, pressure was applied gradually 2 to 4 cms from the tail tip and was increased gradually in intensity until the animal attempted to remove its tail or struggle. The pressure threshold was determined immediately prior to analgesic administration and at suitable intervals thereafter. The analgesic index was calculated using the following formula:

$$\text{Analgesic Index} = \frac{P_{(obs)} - P_{(i)}}{P_{(max)} - P_{(i)}}$$

where $P_{(i)}$ = the pressure threshold before treatment

$P_{(obs)}$ = the pressure threshold after treatment

$P_{(max)}$ = the maximum pressure threshold i.e. 'cut off' point.

It was considered that the pre-treatment pressure threshold was too low in many animals with the result that these rats reacted as soon as the cone was applied implying they were reacting to touch not pain. As the experiments progressed, the animals appeared to become settled and react to a painful stimulus. This observation was upheld by the results from a controlled study from which it was found that the pressure threshold in the control animals increased from the

initial reading upto a plateau after about 0.7 hours. This plateau level of 0.15 of the Analgesic Index Scale was used as the true analgesic threshold in the subsequent experiments.

In the test trials the compounds under test were administered to the rats at a dosage (calculated as morphine base) of 30 mg/kg body weight, the morphine hydrochloride being administered as an aqueous solution and the pamoate and 2-hydroxy-3-naphthoate salts as suspensions in a 1% aqueous solution of sodium carboxymethyl cellulose; the salts employed having a particle size below 180 microns (sieve mesh size 85).

The results of the trials are shown in Figure 1 of the accompanying drawings, from which it may be seen that both the pamoate and 3-hydroxy-2-naphthoate salts have markedly increased periods of true activity as compared with morphine hydrochloride.

In a further trial, morphine pamoate was administered to the test animals at a dosage of 60 mg/kg bodyweight and the results of this trial are shown in Figure 2 of the accompanying drawings.

In human trials, three healthy human volunteers were used to compare the morphine plasma profiles from a morphine suspension and a standard hospital morphine solution.

The standard morphine sulphate solution had the following formula:

| | |
|---|---|
| morphine sulphate | 10 mg |
| absolute alcohol | 1.1 ml |
| syrup BP | 2.5 ml |
| chloroform water | 2.10 ml |

An appropriate volume of the solution was given so that each volunteer received a dose equivalent to 10 mg of morphine base per 70 kg bodyweight.

20 ml of a morphine pamoate suspension was prepared for each volunteer. A dose of 24.4 mg per 70 kg of morphine pamoate was given (equivalent to 13.5 mg of morphine base). The formula for the suspension base was as follows:-

| | |
|---|---|
| sodiumcarboxymethyl cellulose | 1% |
| sodium saccharine | 0.2% |
| rasberry essence | 3% |
| water | ad 100% |

The standard morphine solution was administered on the first day and the morphine pamoate suspension 15 days later.

The average morphine plasma levels for morphine sulphate solution and morphine pamoate suspension for the three volunteers are shown in Figure 3 of the accompanying drawings.

As may be seen, the morphine pamoate suspension gave significant levels of morphine in the plasma over an extended period of time as compared with the morphine suplhate solution and the bioavailability of the morphine from the suspension was generally greater than that from the solution.

Claims:-

1. A salt of morphine with an acid of the formula:

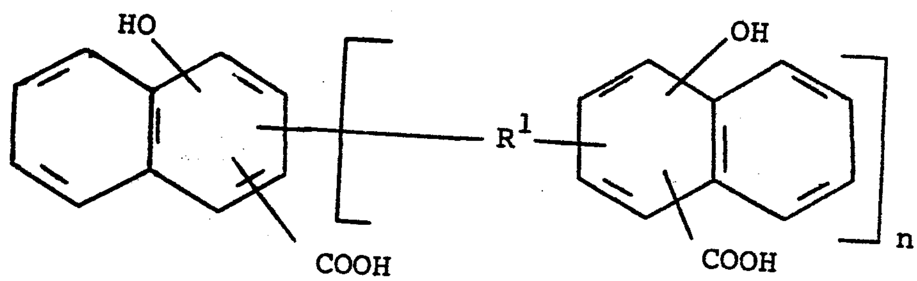

in which $R^1$ is a lower alkylene chain, and _n_ is 0 or 1.

2. A salt of morphine with pamoic acid.

3. Dimorphine pamoate.

4. Morphine 3-hydroxy-2-naphthoate.

5. A pharmaceutical composition comprising a morphine salt as claimed in any one of the preceding claims in association with a pharmaceutical carrier or diluent.

6. A pharmaceutical composition as claimed in claim 5 formulated for oral administration

0137600

1/1

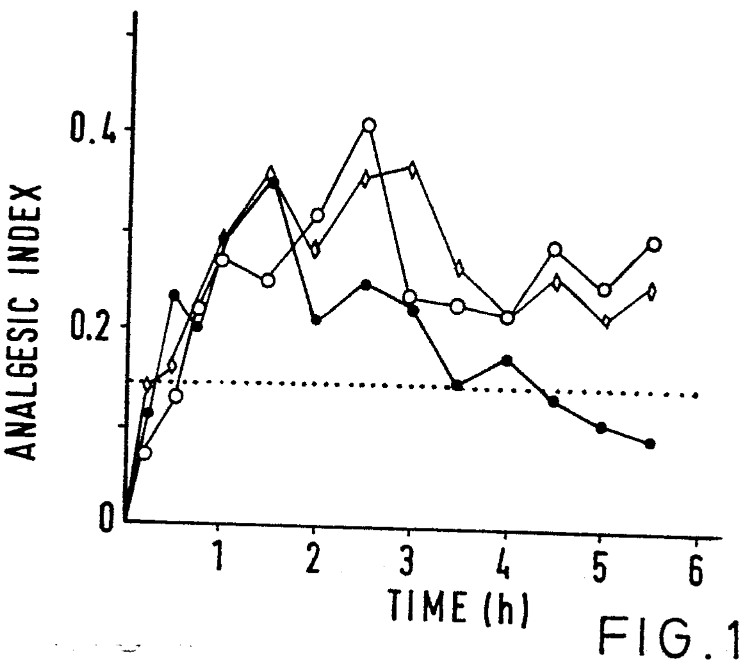


FIG.1.

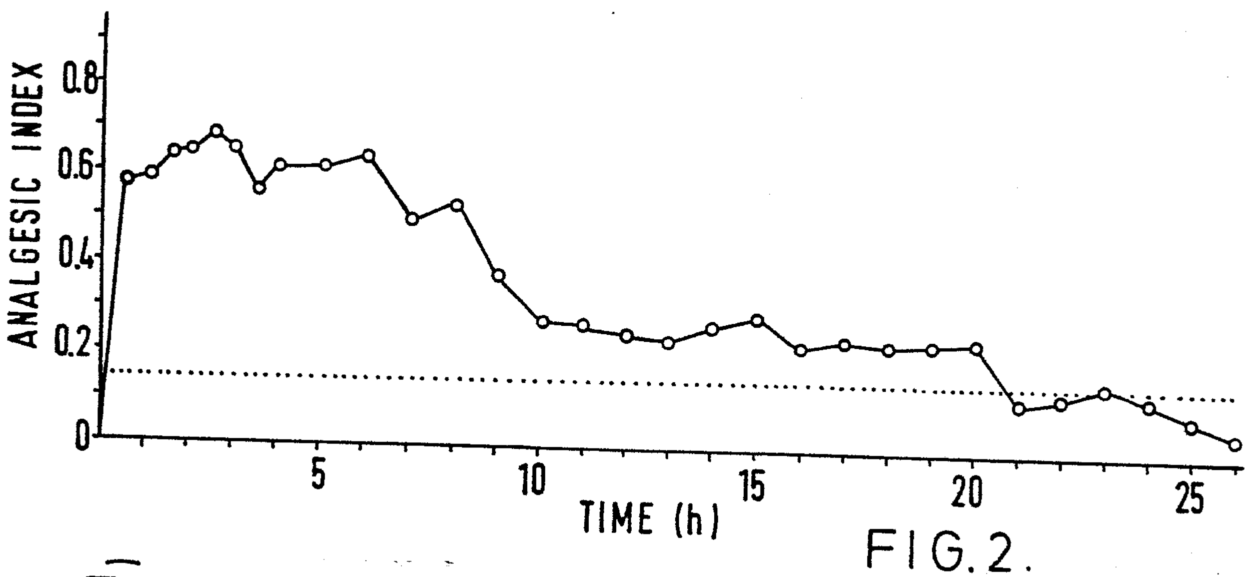


FIG.2.

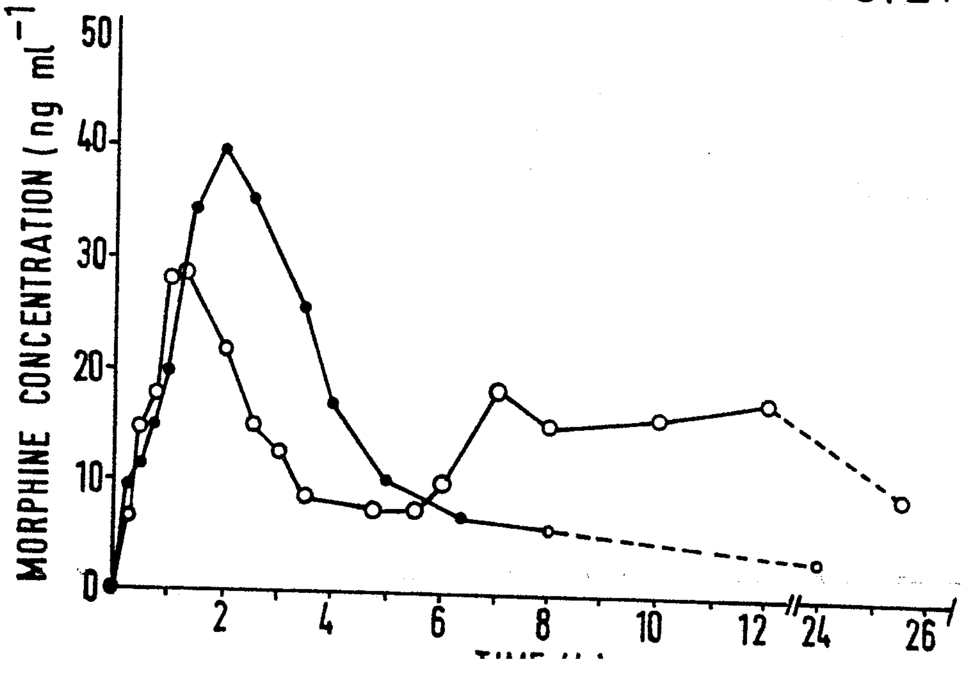


FIG.3.

European Patent Office

**EUROPEAN SEARCH REPORT**

0137600

Application number

EP 84304926.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF PHARMACEUTICAL SCIENCES, vol. 54, 1965<br>W.E. HAMLIN et al. "Relationship between In Vitro Dissolution Rates and Solubilities of Numerous Compounds Representative of Various Chemical Species" pages 1651-1653<br>* Page 1653, especially compound 45, last paragraph *<br>-- | 1,2,5 | C 07 D 489/04<br>A 61 K 31/485 |
| A | FR - A - 1 461 407 (LABORATOIRES SOBIO S.A.)<br>* Claims; page 1; examples 4-6 *<br>-- | 1-3 | |
| A | FR - M - 5 581 (LABORATOIRES SOBIO S.A.)<br>* Page 1 *<br>-- | 1-3,5,6 | TECHNICAL FIELDS SEARCHED (Int. Cl.4)<br>C 07 D 489/00<br>A 61 K 31/00 |
| A | US - A - 3 676 557 (L. LACHMAN et al.)<br>* Claim 1; columns 1,2 *<br>---- | 1-3,5 | |

The present search report has been drawn up for all claims

| Place of search | Date of completion of the search | Examiner |
|---|---|---|
| VIENNA | 25-10-1984 | PETROUSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document